(19) Europäisches Patentamt
European Patent Office
Office européen des brevets

(11) Numéro de publication: 0 295 996
A1

(12) **DEMANDE DE BREVET EUROPEEN**

(21) Numéro de dépôt: 88401311.1

(51) Int. Cl.4: G 06 F 15/20

(22) Date de dépôt: 30.05.88

(30) Priorité: 29.05.87 FR 8707599

(43) Date de publication de la demande: 21.12.88 Bulletin 88/51

(84) Etats contractants désignés: DE FR GB IT NL SE

(71) Demandeur: CENTRE NATIONAL DE LA RECHERCHE SCIENTIFIQUE (CNRS)
15, Quai Anatole France
F-75700 Paris Cedex 07 (FR)

(72) Inventeur: Lumeau, Bernard Jean-François
1, Allée du Lot
31770 Colomiers (FR)

(74) Mandataire: Joly, Jean-Jacques et al
CABINET BEAU DE LOMENIE 55, rue d'Amsterdam
F-75008 Paris (FR)

(54) **Dispositif d'analyse de signaux pour la détection de potentiels évoqués cérébraux.**

(57) Des circuits (14, 16) reçoivent des signaux électriques recueillis au moyen d'un capteur (10) en réponse à une stimulation. Les signaux numérisés sont reçus par un dispositif de calcul (20) pour élaborer un signal représentatif, dans le domaine temporel, de la moyenne d'un petit nombre de signaux de réponse recueillis ; élaborer la transformée de Fourier du signal moyenné ; pour détecter des sinusoïdes exponentiellement amorties contenues dans la transformée de Fourier du signal moyenné et déterminer des paramètres des sinusoïdes détectées, afin de déduire desdits paramètres des informations caractérisant les ondes de potentiel évoqué contenues dans le signal moyenné.

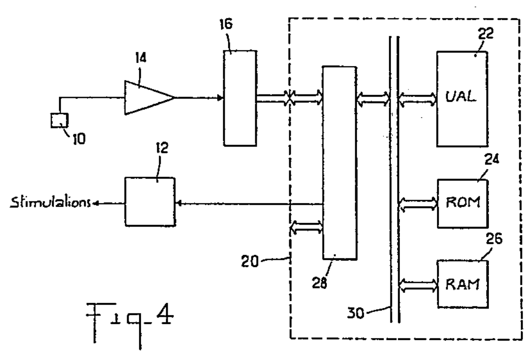


Fig-4

EP 0 295 996 A1

**Description**

## DISPOSITIF D'ANALYSE DE SIGNAUX POUR LA DETECTION DE POTENTIELS EVOQUES CEREBRAUX

La présente invention concerne un dispositif d'analyse de signaux pour le détection de potentiels évoqués cérébraux.

Un potentiel évoqué est la réponse électrique transitoire du système nerveux central à une stimulation. Le potentiel évoqué étant le plus souvent de faible amplitude, il est noyé dans le bruit de fond de l'activité électrique cérébrale spontanée, mais se produit nomalement à une latence fixe par rapport à la stimulation.

L'enregistrement des potentiels évoqués impose de produire en stimulus sensoriel bref, par exemple auditif, d'enregistrer au moyen de capteurs les réponses électriques sur le scalp au niveau de régions du système nerveux central déterminées par l'anatomie des systèmes sensoriels impliqués et d'éliminer les signaux électriques parasites (bruit) se produisant de façon concomitante au potentiel évoqué afin de pouvoir déterminer un certain nombre de caractéristiques du potentiel évoqué, comme, notamment, la latence par rapport à la stimulation, l'amplitude...

L'élimination du bruit pose en problème très difficile en raison du très faible rapport signal à bruit. Dans le cas par exemple de potentiels évoqués auditifs du tronc cérébral (PEATC), le rapport signal à bruit est de l'ordre de -15db.

Une technique actuellement mise en oeuvre pour l'élimination du bruit est celle du moyennage qui met à profit la constance de la latence des potentiels évoqués par rapport à la stimulation. Le moyennage consiste à réaliser un certain nombre de stimulations puis à additionner les réponses obtenues afin de faire émerger le potentiel évoqué par rapport au bruit. Mais la faiblesse du rapport signal à bruit impose un nombre de stimulations élevé, couramment 2000 ou plus dans le cas de PEATC, entraînant de sérieux inconvénients. D'abord la durée des stimulations atteint plusieurs minutes, ce qui est pénible pour le patient. De plus, pour la validité de son interprétation, le résultat obtenu par la technique de moyennage impose la linéarité et la stationarité du milieu cérébral pendant toute la durée des stimulations, hypothèses qui ne sont en fait vérifiées que sur des durées de quelques secondes.

Ainsi, la présente invention a-t-elle pour but de fournir un dispositif d'analyse destiné à la détection des potentiels évoqués et pouvant procurer des résultats fiables sans demander nécessairement la répétition d'un grand nombre de stimulations.

Conformément à l'invention, ce but est atteint grâce à un dispositif comprenant des circuits de réception de signaux électriques recueillis au moyen d'un capteur en réponse à une stimulation et un dispositif de moyennage pour élaborer un signal représentatif, dans le domaine temporel, de la moyenne d'un petit nombre de signaux de réponse recueillis, dispositif dans lequel sont prévus en outre, conformément à l'invention, des moyens de calcul pour élaborer la transformée de Fourier du signal moyenné, et des moyens de traitement pour détecter des sinusoïdes exponentiellement amorties contenues dans la transformée de Fourier du signal moyenné et déterminer des paramètres des sinusoïdes détectées, afin de déduire desdits paramètres des informations caractérisant les ondes de potentiel évoqué des informations caractérisant les ondes de potentiel évoqué contenues dans le signal moyenné.

L'invention repose sur le constat que différentes ondes de potentiels évoqués cérébraux, notamment celles du potentiel évoqué auditif du tronc cérébral, peuvent être modélisées par une courbe représentative d'une somme de fonctions (lorentziennes) dont la transformée de Fourier directe est une sinusoïde exponentiellement amortie. Ainsi la détection et la localisation des potentiels évoqués peuvent alors se ramener à la détermination des paramètres de fréquences pures exponentiellement amorties.

De ces paramètres sont ensuite déduites les caractéristiques recherchées des différentes ondes du potentiel évoqué. Ainsi, pour chaque sinusoïde exponentiellement amortie détectée, on détermine la fréquence, le maximum d'amplitude et le coefficient d'amortissement d'où l'on déduit respectivement la latence, l'amplitude et la largeur à mi-hauteur de l'onde correspondante du potentiel évoqué.

Selon une particularité de l'invention, les moyens de traitement sont agencés pour détecter des sinusoïdes exponentiellement amorties et en déterminer les paramètres dans un signal comportant une composante importante de bruit. De la sorte, il n'est pas nécessaire, comme dans la technique antérieure, de réaliser un moyennage par accumulation d'un grand nombre de stimulations.

En analyse spectrale paramétrique, on connaît des méthodes adaptées à la détermination des paramètres d'une somme de sinusoïdes exponentiellement amorties et de bruit. On pourra en particulier se référer à la méthode décrite par R. Kumaseran et D.W. Tufts dans un article intitulé "Estimating the parameters of exponentially damped sinusoids and pole zero modeling in noise" I.E.E.E. A.S.S.P., Vol. 30, 1982, p. 833-840. Toutefois, cette méthode connue perd de son efficacité lorsque le rapport signal à bruit est faible, ce qui est généralement le cas dans le cadre de mise en oeuvre de l'invention.

Aussi, de préférence, les moyens de traitement du dispositif conforme à l'invention sont agencés pour pouvoir effectuer de façon fiable une détection de sinusoïdes exponentiellement amorties dans un signal fortement bruité.

A cet effet, et comme cela sera décrit de façon plus détaillée par la suite (trois approches différentes sont proposées), les moyens de traitement comprennent des moyens d'estimation d'une matrice de covariance directe, inverse ou directe-inverse (progressive, rétrogradeou progressive-rétrograde) et de séparation efficace des valeurs propres de la matrice liées au bruit.

Il est alors possible de travailler à partir d'un signal moyenné en utilisant un nombre de réponses relativement peu élevé, par exemple moins de 200 au lieu de 2000 comme dans la pratique antérieure.

L'invention sera mieux comprise à la lecture de la description faite ci-après, à titre indicatif mais non limitatif, en référence aux dessins annexés sur lesquels :

- la figure 1 montre schématiquement les différentes ondes du potentiel évoqué auditif du tronc cérébral ;
- la figure 2 illustre la variation dans le temps de la réponse électrique à une stimulation ;
- la figure 3 illustre la variation dans le temps d'un signal obtenu par moyennage des réponses électriques à 128 stimulations ;
- la figure 4 est un schéma par blocs d'un mode de réalisation d'un dispositif d'analyse conforme à l'invention ;
- les figures 5A et 5B sont des organigrammes montant les opérations effectuées par les moyens de traitement du dispositif conforme à l'invention ;
- la figure 6 illustre la séparation des paramètres liés au bruit dans la transformée de Fourier du signal moyenné, séparation telle que le dispositif conforme à l'invention permet de la réaliser ; et
- la figure 7 illustre un exemple de potentiels évoqués estimés respectivement par la technique antérieure et par le dispositif conforme à l'invention.

La figure 1 représente le potentiel évoqué auditif du tronc cérébral. Il est caractérisé par un certain nombre d'ondes notées I à VII. Le praticien s'intéresse généralement aux ondes I à V en recherchant, pour chacune d'entre elles, sa latence $t_i$ (retard entre la stimulation et l'instant où l'on atteint le maximum d'amplitude) et, le cas échéant, son amplitude maximale $a_i$ et sa largeur à mi-hauteur $b_i$, i étant alors un indice variant de 1 à 5. A titre d'exemple, la latence $t_3$, l'amplitude maximale $a_3$ et la largeur à mi-hauteur $b_3$ de l'onde III sont indiquées sur la figure 1.

L'invention est basée sur une modélisation de chaque onde par une courbe représentative d'une fonction particulière du temps, dite lorentzienne, qui s'exprime sous la forme :

$$\frac{a_i}{1 + \frac{(t - t_i)^2}{b_i^2}}, \text{ t étant le temps.}$$

Dans sa partie utile, le potentiel évoqué peut donc être mis sous la forme d'une fonction du temps y (t) telle que :

$$y(t) = \sum_{i=1}^{5} \frac{a_i}{1 + \frac{(t-t_i)^2}{b_i^2}}.$$

Comme déjà indiqué, la détection du potentiel évoqué est rendue particulièrement difficile en raison du bruit dans lequel il est noyé. La figure 2 représente un exemple de réponse électrique à une stimulation ; il est impossible d'y reconnaître les ondes de la figure 1.

La technique de moyennage permet une amélioration du rapport signal à bruit, mais son emploi n'est légitime que si les hypothèses de linéarité et de stationnarité du milieu cérébral sont vérifiées pendant toute la durée des stimulations. Cette durée doit alors être limitée à quelques secondes, ce qui limite en pratique le nombre de stimulations à moins de 200. La figure 3 illustre la variation dans le temps du signal obtenu après moyennage de 128 réponses. Ce signal ne peut être exploité directement pour rechercher les caractéristiques des ondes du potentiel évoqué, ondes que l'on peut simplement deviner. Il est donc nécessaire d'effectuer un traitement spécifique du signal moyenné.

A titre indicatif, la courbe A de la figure 7 illustre un potentiel évoqué estimé après un moyennage effectué sur 2048 stimulations conformément à la pratique antérieure. Une exploitation directe de la courbe A est certes possible, mais sans garantie de fiabilité du résultat et au prix d'une épreuve pénible pour le patient.

La figure 4 est un schéma par blocs d'un dispositif d'analyse conforme à l'invention.

Une électrode de scalp 10 permet de recueillir les réponses électriques à des stimulations auxquelles un patient est soumis, par exemple des stimulations auditives constituées par des sons brefs produits avec une fréquence de répétition de plusieurs hertz au moyen d'un stimulateur 12.

Un signal recueilli par l'électrode 10 (par exemple du type de celui illustré par la figure 2) est amplifié par un amplificateur 14 puis est converti sous forme numérique au moyen d'un convertisseur analogique-numérique 16. L'information numérique obtenue est reçue par un circuit de traitement 20.

De façon classique, le circuit de traitement 20 comprend une unité arithmétique et logique 22, des circuits de mémoire morte (ROM) 24 et vive (RAM) 26 et des circuits d'entrée/sortie 28 interconnectés par bus 30. La commande du stimulateur 12 est assurée par des signaux produits par l'unité de traitement afin de synchroniser les stimulations et les origines des temps des réponses électriques recueillies.

Le circuit de traitement 20 fonctionne sous la commande de programmes stockés en mémoire morte. Les opérations réalisées par le circuit de traitement sont indiquées sur les organigrammes des figures 5A à 5B.

Les phases successives de traitement sont indiquées de façon générale sur la figure 5A. Elles consistent dans des opérations de :

- moyennage des réponses électriques à un nombre N de stimulations (phase 51),
- transformation de Fourier du signal moyenné (phase 52),
- détection et détermination des paramètres d'exponentielles complexes dans la transformée de Fourier du signal moyenné (phase 53), et
- détermination des caractéristiques des ondes du potentiel évoqué (phase 54).

Le moyennage est effectué en cumulant successivement les informations numériques correspondant aux signaux électriques recueillis en réponse aux différentes stimulations. Comme déjà indiqué, l'invention peut être mise en oeuvre en utilisant un nombre de stimulations relativement peu élevé, par exemple moins de 200.

La transformation de Fourier est une opération classique. La modélisation des ondes du potentiel évoqué par des courbes lorentziennes fait que ces ondes se retrouvent sous forme de sinusoïdes exponentiellement amorties dans la transformée de Fourier du signal moyenné.

L'opération 53 de détection et de détermination des paramètres de sinusoïdes exponentiellement amorties dans la transformée de Fourier du signal moyenné comprend les phases indiquées dans les organigrammes de la figure 5B. Ces organigrammes correspondent à la mise en oeuvre de méthodes originales permettant de détecter de façon fiable sinusoïdes exponentiellement amorties noyées dans un bruit important.

Dans une première phase, selon une première approche (figure 5Ba), la matrice de covariance est estimée en cumulant les covariances élémentaires de P vecteurs progressifs et P vecteurs rétrogrades de dimension M. Chaque vecteur progressif est formé de M valeurs successives de la transformée de Fourier directe du signal moyenné, l'ensemble des P vecteurs progressifs étant obtenu par décalage successif d'un échantillon de la transformée de Fourier directe. Les vecteurs rétrogrades sont obtenus en retournant et en conjuguant les composantes des vecteurs progressifs. Pour s'assurer du fait que la matrice soit de rang M on doit utiliser un nombre total de vecteurs tel que $2\,P \geqq M$. Pour identifier R exponentielles complexes on doit choisir une dimension M supérieure à $(2\,R) + 1$, 2 R étant la dimension de l'espace engendré par les R exponentielles complexes et les R exponentielles complexes rétrogrades associées (phase 531).

La matrice de covariance est diagonalisée, la diagonalisation est une opération classique. Les 2 R plus grandes valeurs propres sont sélectionnées comme correspondant à l'espace signal. Celui-ci est identifié comme l'espace engendré par les 2 R vecteurs propres qui leur sont associés. Par exemple, dans le cas des PEATC, R est égal à 5 (phase 532).

Un vecteur $\underline{A}$ est choisi orthogonal à l'espace signal de telle sorte que si K, $Kz_i$, $Kz_i^2 + \ldots, Kz_i^{M-1}$ est l'un quelconque des signaux à identifier, l'orthogonalité avec $\underline{A}$ entraîne :

$a_0 + a_1 z_i + \ldots + a_{M-1}\, z_i^{M-1} = 0$,

avec $z_i = \exp(\delta_i + 2\pi j f_i)$ où $\delta_i$ est l'amortissement et $f_i$ la fréquence de la $i^{ème}$ exponentielle complexe.

C'est-à-dire que $z_i$ est zéro du polynôme A (z) associé à $\underline{A}$ :

$A(z) = a_0 + a_1 z + a_2 z^2 + \ldots + a_{M-1} z^{M-1}$.

Si $M > (2\,R) + 1$, il existe une infinité de vecteurs orthogonaux à l'espace signal. De façon originale, on introduit un critère de choix (norme minimale et contrainte de normalisation) qui permet de minimiser les erreurs dues au bruit résiduel, ce qui conduit à choisir la solution :

$$\underline{A} = A_0\, e^{j\varphi} + \underline{\tilde{A}}_0\, e^{-j\varphi}$$

où

$$\underline{A}_0 = \sum_{i=2R+1}^{M} u^*_{1i}\, \underline{U}_i$$

$\varphi = 1/2$ argument $(A_0^+ \tilde{A}_0)$

et où $\underline{\tilde{A}}_0$ désigne le vecteur obtenu en inversant l'ordre des composantes de $\underline{A}_0$ et en conjuguant, $\underline{U}_i$ désigne le $i^{ème}$ vecteur propre normé de la matrice de covariance et $u^*_{1i}$ désigne la première composante du vecteur $\underline{U}_i$. La somme définissant $A_0$ porte bien sur les M -2 R derniers vecteurs propres (correspondant aux plus petites valeurs propres), tous orthogonaux à l'espace signal (phase 533).

Le choix d'un vecteur $\underline{A}$ orthogonal à l'espace signal assure, comme indiqué ci-dessus, que les paramètres $z_i$ caractéristiques des exponentielles figurent parmi les racines du polynômes A (z) associé à $\underline{A}$. Grâce au mode original de choix du vecteur $\underline{A}$ décrit ci-dessus, on assure que l'ensemble des racines du polynôme est réparti de la façon suivante :

- les R racines à déterminer $z_i$, de module $|z_i| < 1$,
- les R racines $z'_i$ associées au précédentes par la relation $z'_i = 1/z_i^*$ de module $|z'_i| > 1$,
- les M - 2 R - 1 racines parasites supplémentaires de module égal à 1. La répartition des racines ainsi obtenue est indiquée figure 6 (LUM - CLE). Elle permet donc d'identifier sans ambiguité parmi les M - 1 racines du polynôme A (z) les paramètres $z_i$ comme étant les R racines de module strictement inférieur à un, ceux liés au bruit étant sur le cercle de rayon unité (phase 534).

Les paramètres des exponentielles se déduisent de façon classique des paramètres complexes $z_i$ : amortissement $|z_i|$, fréquence argument $(z_i)$ ; et les amplitudes s'en déduisent par une méthode de moindres carrés (phase 535).

La deuxième approche relative à la figure 5B(b) consiste, à partir de la matrice de covariance estimée en cumulant les covariances élémentaires de P vecteurs rétrogrades de dimension M, à tenir compte dans l'estimation du fait que le rapport signal sur bruit moyen varie d'une ligne à une autre sur la matrice formée des P vecteurs de dimensions M. Dès lors, avant d'appliquer la méthode décrite par R. Kumaseran et D. W. Tufts signalée plus haut, chaque ligne de la matrice des P vecteurs de dimension M est pondérée en fonction des coefficients d'amortissement des exponentielles amorties à estimer (phase 536). Comme ces coefficients sont inconnus, la méthode est itérative : aucune pondération lors de la première estimation, pondération en fonction des coefficients d'amortissement estimés, pour la deuxième estimation, et rebouclage (phase 532) jusqu'à convergence. La répartition des racines ainsi obtenue est indiquée figure 6 (LUM - LEV). On observe que ces racines se trouvent à l'intérieur du cercle unité.

La figure 5B (c) schématise une approche différente dans le principe des deux précédentes. Après la phase d'estimation de la matrice de covariance (celle-ci pouvant se faire soit à partir de P vecteurs progressifs, soit de P vecteurs rétrogrades), la matrice de covariance "débarrassée" du bruit de dimension (M, M) et son rang R sont estimés, comme indiqué dans la demande de brevet français No 87 15 368 relative à un "Dispositif de stéréolocalisation spatiale de sources d'activités cérébrales" (phase 537). Les vecteurs sources formés par l'ensemble des $\{z_i\}$ et caractérisant l'espace signal sont invariants par translation. Dans ces conditions, il est possible de former à partir de la matrice (M, M) une matrice de covariance de dimension (R + 1, R + 1) et de rang R (phase 538). La matrice ainsi formée étant "débarrassée" du bruit et d'une unité supérieure à son rang, le vecteur de l'espace bruit, orthogonal à l'espace signal, qui est recherché pour identifier les fréquences et amortissements caractéristiques des vecteurs source, est associé à la valeur propre minimale de la matrice de covariance de dimension (R + 1, R + 1) (phase 579). Dès lors, on est ramené à la phase 534. La répartition des racines ainsi obtenues est indiquée figure 6 (LUM = PIS). On observe que ces racines se trouvent à l'intérieur du cercle unité.

Un mode de réalisation d'un dispositif selon l'invention a été décrit ci-avant dans le cadre d'une application à la caractérisation de potentiels évoqués auditifs du tronc cérébral. Toutefois, l'invention est bien entendu applicable à la caractérisation d'autres potentiels évoqués dès lors qu'ils présentent des formes d'ondes pouvant être modélisées par des lorentziennes noyées dans du bruit.

**Revendications**

1. Dispositif d'analyse de signaux pour la détection de potentiels évoqués, notamment de potentiels évoqués cérébraux, comportant des circuits (10, 14) de réception de signaux électriques recueillis au moyen d'un capteur en réponse à une stimulation et un dispositif de moyennage pour élaborer un signal représentitif, dans le domaine temporel, de la moyenne de signaux de réponse recueillis, des ondes de potentiel évoqué contenu dans le signal moyenné pouvant se modéliser par une somme de lorentziennes noyées dans du bruit, caractérisé en ce que sont prévus en outre : des moyens de calcul pour élaborer la transformée de Fourier du signal moyenné, et des moyens de traitement pour détecter des sinusoïdes exponentiellement amorties contenues dans la transformée de Fourier du signal moyenné et déterminer des paramètres des sinusoïdes détectées, afin de déduire desdits paramètres des informations caractérisant les ondes (I à V) de potentiel évoqué contenues dans le signal moyenné, celui-ci étant formé à partir d'un nombre relativement petit de signaux de réponse recueillis.

2. Dispositif selon la revendication 1, caractérisé en ce que les moyens de traitement comprennent des moyens pour successivement : estimer une matrice de covariance directe, inverse ou directe-inverse à partir des valeurs de la transformée de Fourier directe du signal moyenné ; diagonaliser la matrice de covariance pondérée ou non, ou diagonaliser la matrice de covariance "débarrassée" du bruit après traitement et sélectionner les plus grandes valeurs propres comme étant celles liées à l'espace signal ; sélectionner un vecteur orthogonal à l'espace signal satisfaisant à une condition de norme minimale sous contrainte ou étant associé à la valeur propre minimale de la matrice de covariance obtenue à partir de la matrice de covariance "débarrassée" du bruit, de manière à inposer aux racines liées au bruit d'un polynôme associé au vecteur sélectionné de se trouver sur le cercle unité ou à l'intérieur du cercle unité, et rechercher les racines dudit polynôme associé au vecteur sélectionné qui caractérisent des exponentielles amorties.

3. Dispositif selon la revendication 1, caractérisé en ce que les moyens de traitement sont agencés pour calculer l'un au moins des paramètres consistant dans la latence, l'amplitude et la largeur à mi-hauteur des ondes du potentiel évoqué à partir, respectivement, de la fréquence, du maximum d'amplitude et du coefficient d'amortissement des exponentielles amorties détectées.

4. Dispositif selon l'une quelconque des revendications 1 à 3, destiné à la caractérisation des ondes des potentiels évoqués auditifs du tronc cérébral.

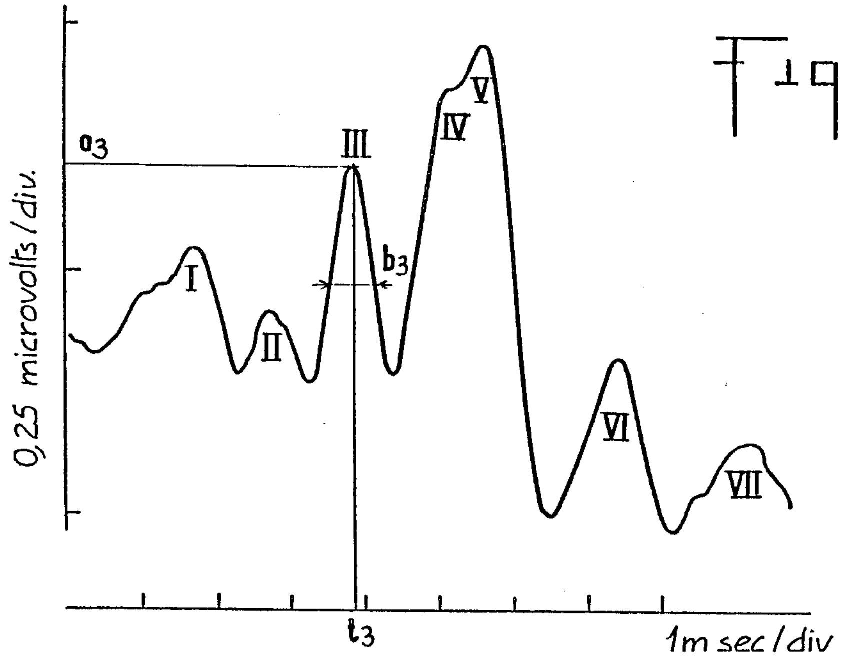
a3
III
IV
V
I
II
b3
VI
VII
0,25 microvolts / div.
t3
1m sec / div

Fig-1

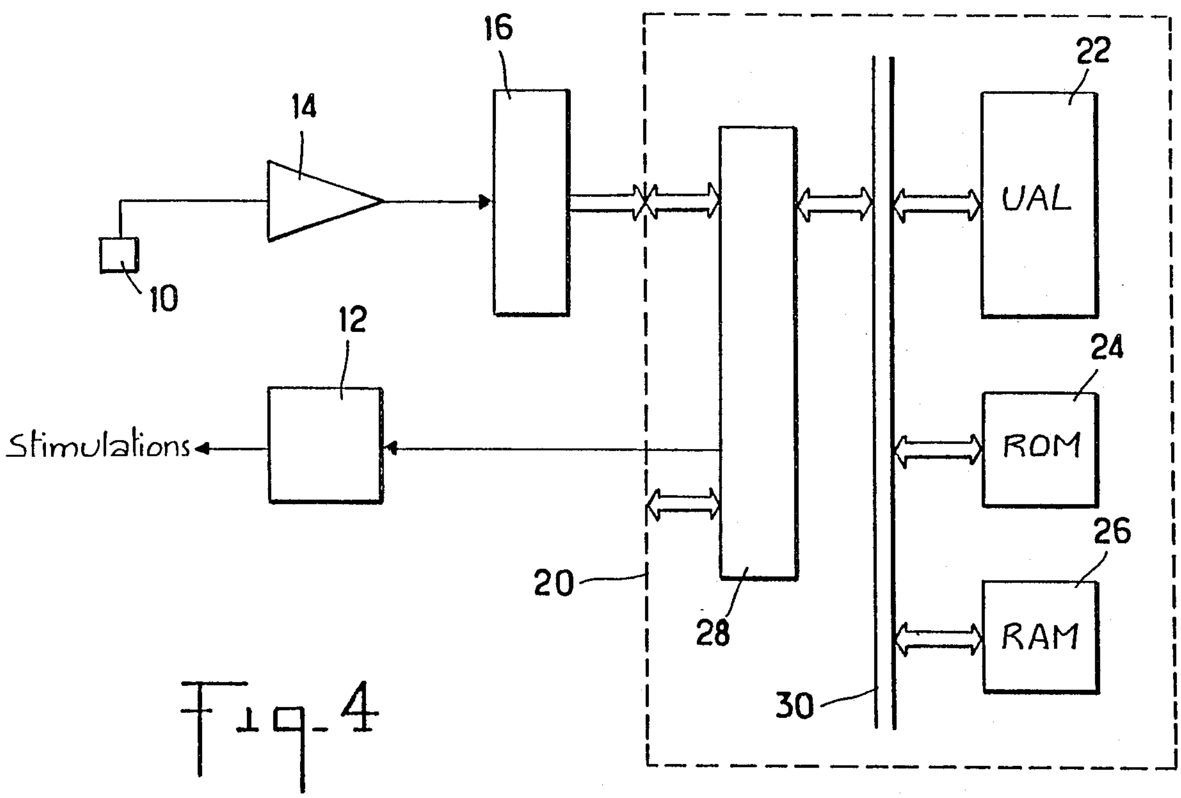
16
14
22
UAL
10
12
24
ROM
Stimulations
26
RAM
20
28
30

Fig-4

Fig 2

Réponse à une stimulation

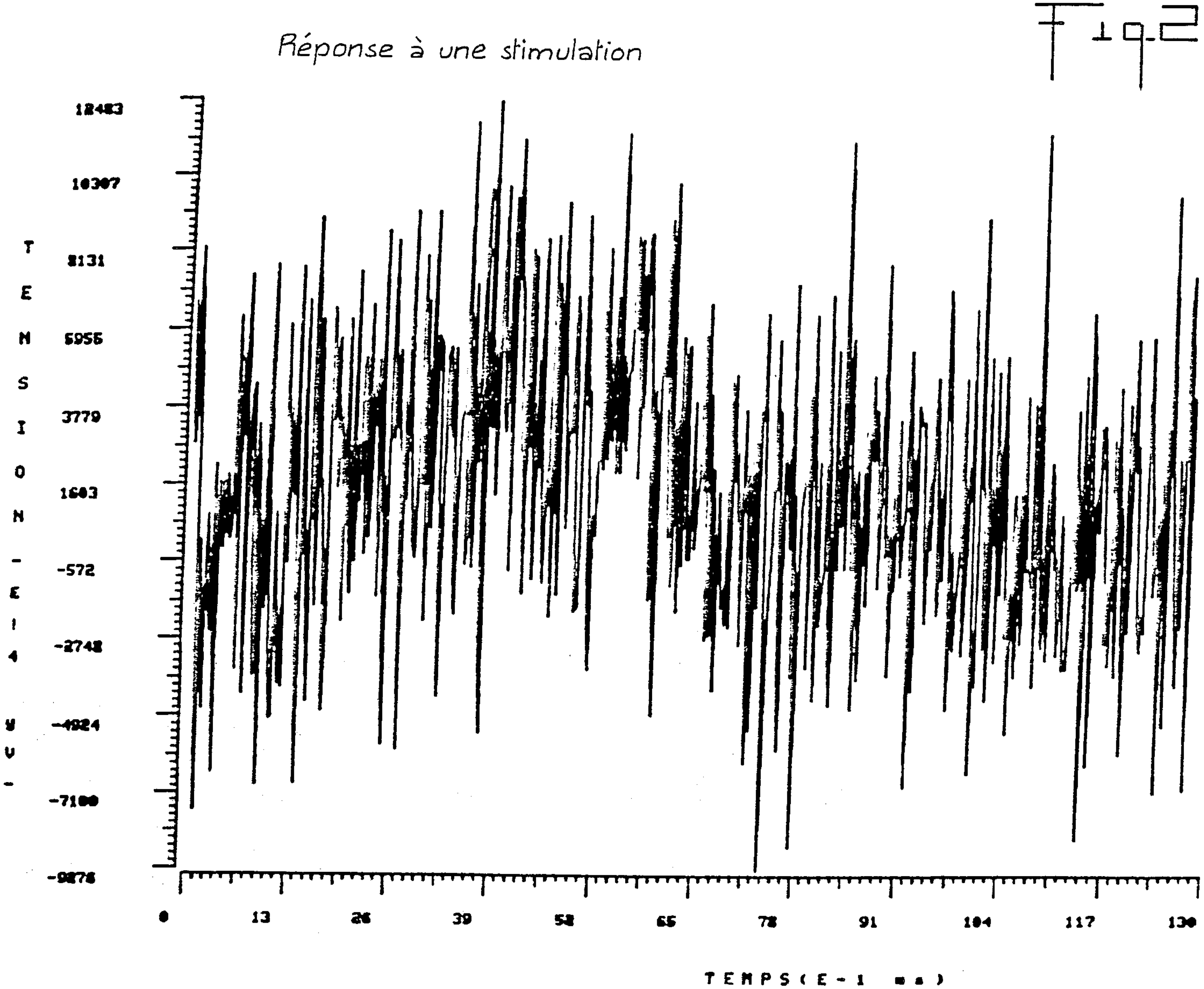

Fig.3

Potentiel évoqué auditif du tronc cérébral après moyennage de 128 reponses

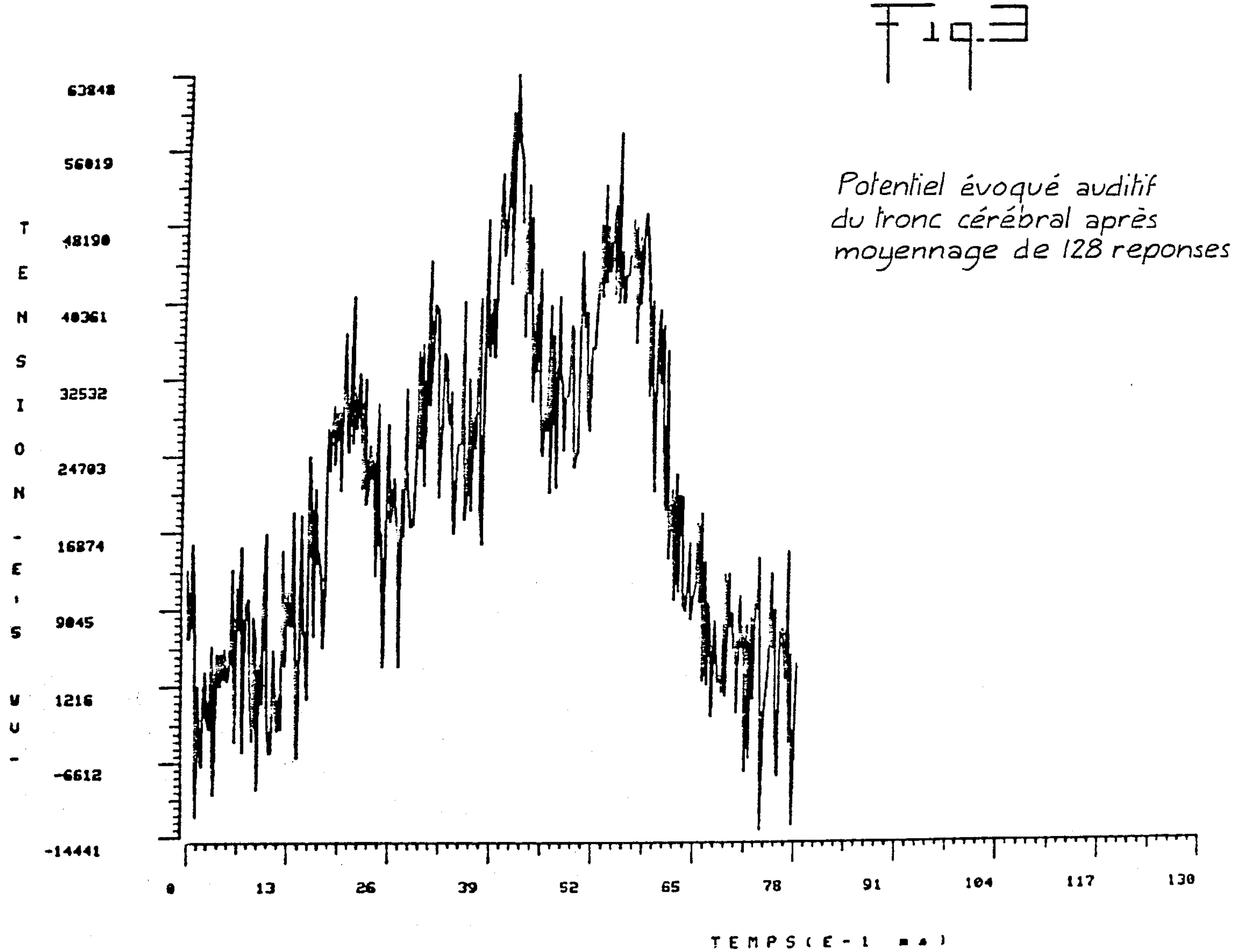

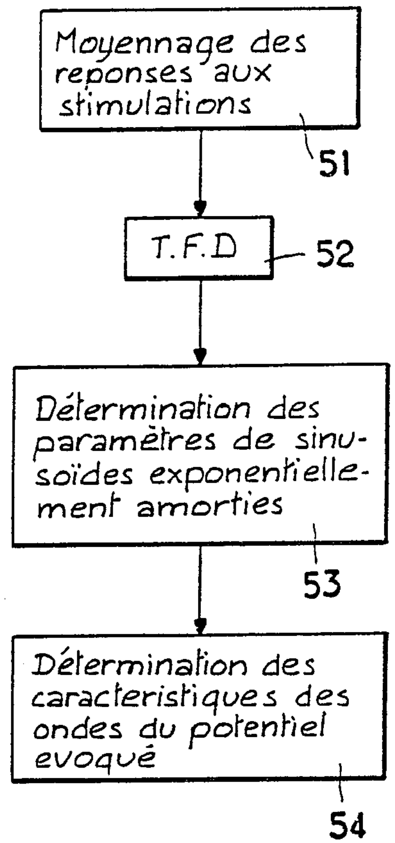
Moyennage des reponses aux stimulations
51
T.F.D
52
Détermination des paramètres de sinusoïdes exponentiellement amorties
53
Détermination des caracteristiques des ondes du potentiel evoqué
54

Fig.5A

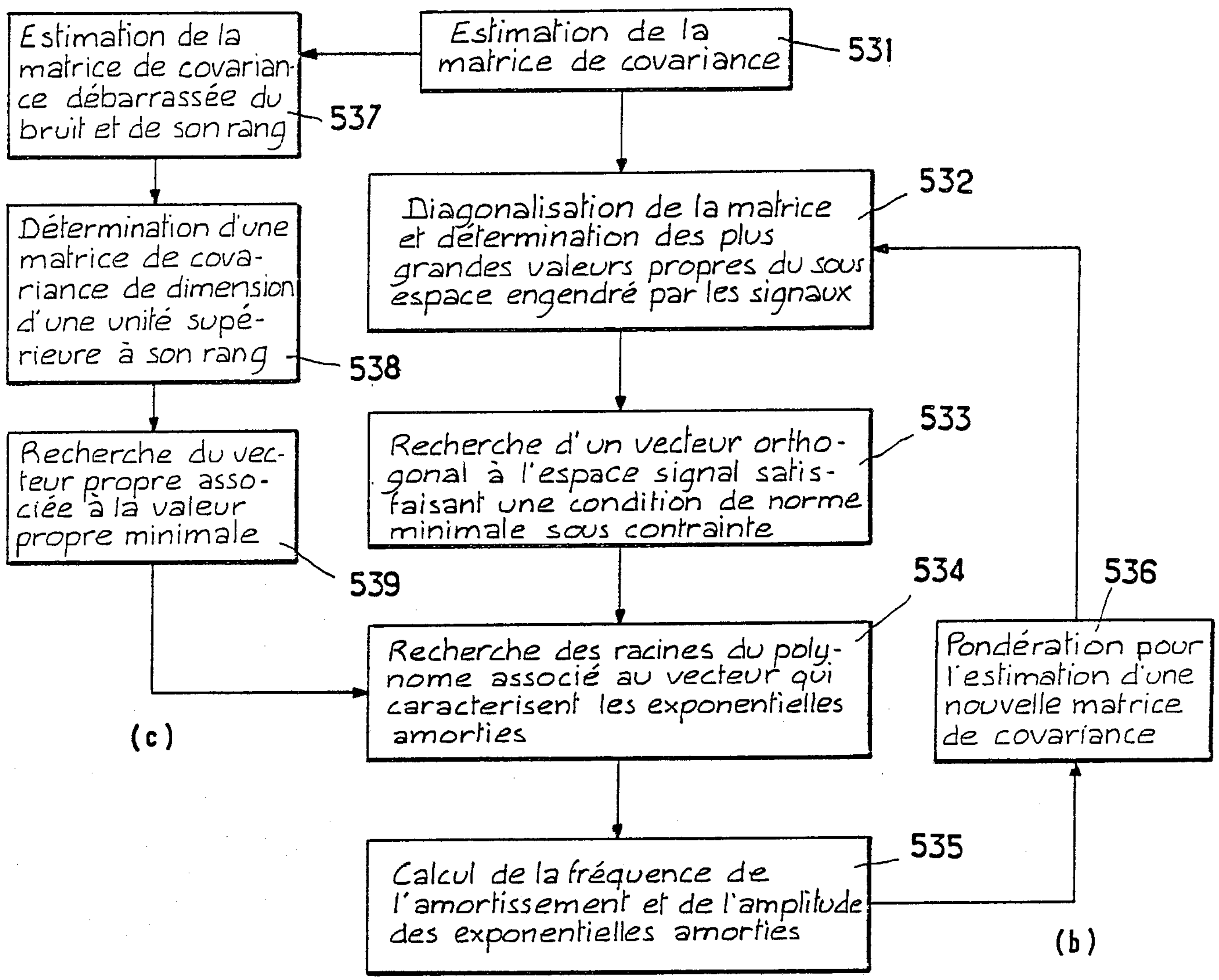
Estimation de la matrice de covariance
531
Estimation de la matrice de covariance débarrassée du bruit et de son rang
537
Diagonalisation de la matrice et détermination des plus grandes valeurs propres du sous espace engendré par les signaux
532
Détermination d'une matrice de covariance de dimension d'une unité supérieure à son rang
538
Recherche d'un vecteur orthogonal à l'espace signal satisfaisant une condition de norme minimale sous contrainte
533
Recherche du vecteur propre associée à la valeur propre minimale
539
534
536
Recherche des racines du polynome associé au vecteur qui caracterisent les exponentielles amorties
Pondération pour l'estimation d'une nouvelle matrice de covariance
(c)
Calcul de la fréquence de l'amortissement et de l'amplitude des exponentielles amorties
535
(b)
(a)

Fig.5B

Fig.6

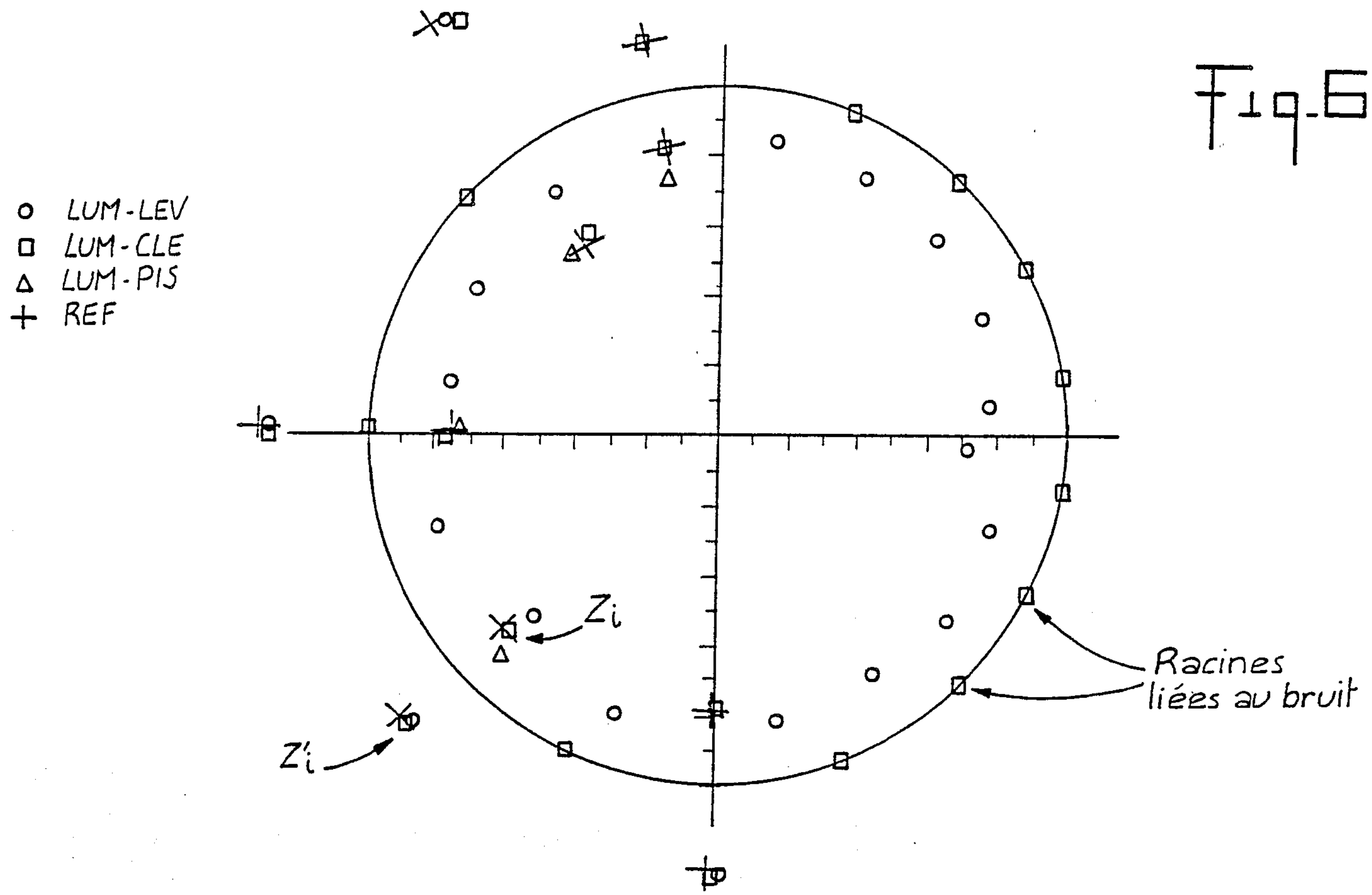

Fig. 7

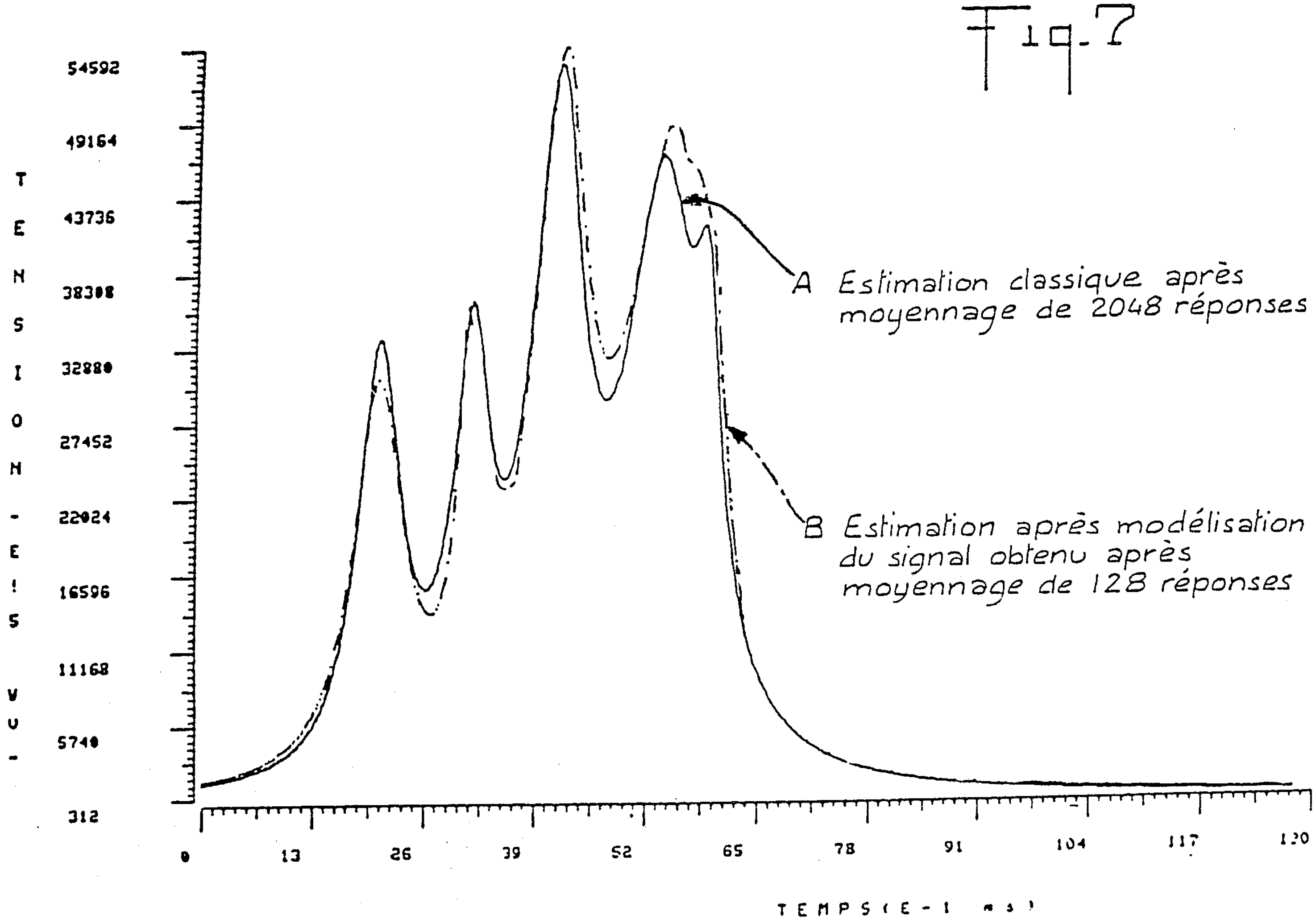
TENSION-E!S VU-
54592
49164
43736
38308
32880
27452
22024
16596
11168
5740
312
0
13
26
39
52
65
78
91
104
117
120
TEMPS(E-1 ms)
A Estimation classique après moyennage de 2048 réponses
B Estimation après modélisation du signal obtenu après moyennage de 128 réponses

Office européen des brevets

# RAPPORT DE RECHERCHE EUROPEENNE

Numero de la demande

EP 88 40 1311

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | WO-A-8 203 977 (DUFFY)<br>* Page 10, ligne 4 - page 12, ligne 3; page 54, lignes 1-13 *<br>--- | 1,2 | G 06 F 15/20 |
| A | IEEE TRANSACTIONS ON BIOMEDICAL ENGINEERING, vol. BME-32, no. 12, décembre 1985, pages 1066-1070, IEEE, New York, US; J.R. BOSTON: "Noise cancellation for brainstem auditory evoked potentials"<br>* Résumé: "Introduction" *<br>--- | 1 | |
| D,A | IEEE TRANSACTIONS ON ACOUSTICS, SPEECH, AND SIGNAL PROCESSING, vol. ASSP-30, no. 6, décembre 1982, pages 833-840, IEEE, New York, US; R. KUMARESAN et al.: "Estimating the parameters of exponentially damped sinusoids and pole-zero modeling in noise"<br>----- | | |

DOMAINES TECHNIQUES RECHERCHES (Int. Cl.4)

G 06 F 15/20

Le présent rapport a été établi pour toutes les revendications

| Lieu de la recherche | Date d'achèvement de la recherche | Examinateur |
|---|---|---|
| LA HAYE | 05-09-1988 | CHUGG D.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)